# FASCICULE DE BREVET EUROPEEN

(11) **EP 3 209 284 B1**
(45) Date de publication et mention de la délivrance du brevet: **14.11.2018**
(21) Numéro de dépôt: 15709722.1
(22) Date de dépôt: 20.02.2015
(51) Int. Cl.: A61P 31/18, A61K 36/47, A61K 9/14, A61K 31/00

(54) **COMPOSITION À BASE D'ANTHOSTEMA SENEGALENSE UTILISÉE COMME MÉDICAMENT CONTRE LE SIDA**
AUF ANTHOSTEMA SENEGALENSE BASIERENDE ZUSAMMENSETZUNG ZUR VERWENDUNG ALS ARZNEIMITTEL GEGEN AIDS
ANTHOSTEMA SENEGALENSE-BASED COMPOSITION, FOR USE AS AN ANTI-AIDS DRUG

(30) Priorité: 10.06.2014 FR 1455226
(43) Date de publication de la demande: 30.08.2017
(73) Titulaire: Laboratoire Michel Iderne, 67560 Rosheim (FR); Balde, Aliou Mamadou, 1150 Bruxelles (BE)
(72) Inventeur: BALDE, Aliou Mamadou, B-1150 Bruxelles (BE)
(74) Mandataire: Cabinet Bleger-Rhein-Poupon
(86) Numéro de dépôt international: PCT/FR2015/050413
(87) Numéro de publication internationale: WO 2015/189487

(56) Documents cités:
- ABREU P.M. ET AL.: "Antimicrobial, antitumor and antileishmania screening of medicinal plants from Guinea-Bissau.", PHYTOMEDICINE, vol. 6, no. 3, 1999, pages 187-195, XP002733515,
- DATABASE BIOSIS [Online] BIOSCIENCES INFORMATION SERVICE, PHILADELPHIA, PA, US; janvier 2005 (2005-01), KONE W MAMIDOU ET AL: "Anthelmintic activity of medicinal plants used in northern Cote d'Ivoire against intestinal helminthiasis", XP002733516, Database accession no. PREV200510007676 & KONE W MAMIDOU ET AL: "Anthelmintic activity of medicinal plants used in northern Cote d'Ivoire against intestinal helminthiasis", PHARMACEUTICAL BIOLOGY, vol. 43, no. 1, janvier 2005 (2005-01), pages 72-78, ISSN: 1388-0209
- NIKIÉMA J.B. ET AL.: "Stratégie d'utilisation des substances naturelles dans la prise en charge des personnes vivant avec le VIH ; expérience du Burkina Faso.", ETHNOPHARMACOLOGIA, no. 43, juillet 2009 (2009-07), pages 47-51, XP002733517,
- BALDE A M ET AL: "The Guinean traditional medicine in the treatment of HIV/AIDS", RETROVIROLOGY, vol. 3, no. Suppl. 1, 2006, page P6, XP002733518, & INTERNATIONAL MEETING OF THE INSTITUTE-OF-HUMAN-VIROLOGY; BALTIMORE, MD, USA; NOVEMBER 17 -21, 2006

## Description

La présente invention concerne l'utilisation d'*Anthostema senegalense* dans la préparation d'un médicament pour le traitement de l'infection par le Virus de l'Immunodéficience Humaine (VIH), du sida (syndrome de l'immunodéficience acquise) et des manifestations cliniques qui l'accompagnent.

Le syndrome de l'immunodéficience acquise, plus connu sous son acronyme sida, est une maladie se traduisant par un ensemble de symptômes consécutifs à la destruction de cellules du système immunitaire par un rétrovirus appelé virus de l'immunodéficience humaine ou VIH, dont on connait actuellement deux types : VIH-1 et VIH-2. Le sida est le dernier stade de l'infection par ce virus, qui conduit généralement à la mort des personnes infectées des suites de maladies opportunistes.

La transmission du virus du Sida se fait par voie sexuelle, par voie sanguine, ou par transmission de la mère à l'enfant (à travers le placenta ou lors l'accouchement et/ou de l'allaitement). Les particules virales de VIH présentes dans les fluides corporels viennent infecter les cellules du système immunitaire, en particulier les lymphocytes T4 qui possèdent des récepteurs adéquats (les protéines CD4).

A la suite de la primo-infection, le patient traverse une phase asymptomatique pouvant durer une dizaine d'années, durant laquelle il ne présente aucun symptôme de la maladie mais pendant laquelle ses lymphocytes T4 et ses macrophages sont progressivement détruits par le VIH. À ce stade, l'individu possède des anticorps dirigés contre le virus: il est dit séropositif.

Lorsque son système immunitaire est trop affaibli, les symptômes du Sida apparaissent. Des maladies opportunistes se déclarent, comme par exemple la tuberculose, des pneumonies ou des pneumocystoses, mais aussi des herpès, ou des cancers (sarcome de Kaposi, lymphomes...) pouvant conduire à la mort du patient.

L'infection par le virus du VIH est une pandémie qui s'est développée à partir de la fin des années 1970 et qui frappe maintenant la planète entière, aussi bien les pays développés que les pays en voie de développement, faisant de cette maladie un problème sanitaire mondial. Selon une estimation de l'Organisation Mondiale de la Santé (OMS) datant de décembre 2012, plus de 34 millions de personnes seraient infectées par le virus du VIH dans le monde, dont 3,3 millions d'enfants de moins de 15 ans. L'Afrique subsaharienne rassemblerait à elle seule 69 % des cas. On compterait environ 2,5 millions de nouvelles contaminations chaque année, et 1,7 millions de décès auraient été recensés en 2011.

Actuellement, il n'est pas possible de guérir du Sida et la prévention, notamment l'usage du préservatif, reste la meilleure option pour lutter contre le virus du VIH, car il n'existe pour l'instant aucun vaccin permettant de se protéger de ce virus.

Il existe néanmoins des traitements qui permettent de ralentir l'évolution de la maladie vers le stade Sida. Ces traitements consistent à prendre une ou préférentiellement plusieurs molécules antivirales en association pour plus d'efficacité. Les trithérapies, qui associent trois molécules antivirales, sont généralement préconisées et sont considérées comme les plus efficaces pour contenir l'action du virus et stopper sa prolifération au sein de l'organisme.

Ces traitements, qui doivent être poursuivis pendant toute la vie des patients, sont très coûteux et ne sont de ce fait que très rarement accessibles dans les pays en voie de développement car les populations ne peuvent pas en assurer la charge financière. Dans ces pays, notamment en Afrique et en Asie où le virus est très présent, une très grande majorité des patients ne bénéficie aujourd'hui d'aucun traitement efficace. Or, en l'absence de traitement, la quasi-totalité des patients infectés par le VIH évolue vers le sida, puis la mort.

En outre, même dans les pays où les trithérapies sont prescrites, celles-ci ne sont pas efficaces sur tous les patients. Il existe en effet une part non négligeable de patients traités pour lesquels ce type de traitement reste peu efficace (échappement virologique), voire même complètement inefficace (échec thérapeutique).

Par ailleurs, même lorsqu'ils sont efficaces, ces traitements présentent des effets indésirables importants, ainsi qu'une certaine toxicité, qui amoindrit la qualité de vie des patients. Certains patients, ne supportant plus ces effets secondaires, stoppent leur traitement, alors que celui-ci doit au contraire être suivi avec une très grande régularité pour éviter de rendre le virus résistant.

Enfin, les molécules utilisées dans les trithérapies sont souvent des molécules fragiles qui nécessitent des conditions strictes de conservation, ce qui est particulièrement problématique dans les pays chauds et/ou humides notamment en Afrique et en Asie.

Dans les pays en développement, notamment en Afrique, il serait particulièrement intéressant pour toutes ces raisons, de disposer d'un traitement efficace et beaucoup moins cher, obtenu à partir de plantes locales et sous une forme ne posant pas de problèmes de conservation.

C'est le but recherché par l'invention dont l'objectif est de fournir un médicament efficace, générant moins d'effets secondaires et dont le coût resterait faible par rapport au coût des trithérapies préconisées actuellement en cas d'infection par le VIH.

Ce médicament pourrait également être proposé dans les pays développés, en complément des trithérapies actuelles pour une meilleure efficacité ou pour traiter des effets secondaires indésirables, ou en remplacement de celles-ci pour des raisons économiques, pour limiter les effets secondaires ou dans les cas où les traitements classiques sont inefficaces.

Pour résoudre ce problème technique, l'invention enseigne une composition pharmaceutique à base d'*Anthostema senegalense* pour son utilisation comme médicament dans le traitement de l'infection par le Virus de l'Immunodéficience Humaine (VIH), du sida et des manifestations cliniques qui l'accompagnent.

Cette composition pharmaceutique peut être utilisée comme médicament antirétroviral contre le virus VIH de type 1 ou de type 2 Avantageusement, la composition selon l'invention ne présente pas ou très peu d'effets secondaires et est beaucoup moins chère que les médicaments classiques contre le sida. Elle est ainsi beaucoup mieux acceptée par les patients pour un traitement régulier de longue durée.

De plus, son effet est renforcé par les autres composants présents dans la plante qui se trouvent mélangés avec le principe actif lorsque l'on réalise la composition thérapeutique. Le totum présente ainsi un effet amélioré par rapport au principe actif à l'état isolé.

En outre et comme cela est fréquent avec les extraits naturels de plantes non modifiés chimiquement, le principe actif de la composition pharmaceutique de l'invention présente de très faibles risques de surdosage.

D'autres caractéristiques et avantages de l'invention apparaîtront à la lecture de la description détaillée qui va suivre.

*Anthostema senegalense* est une plante à fleur de la famille des Euphorbiacées.

Il s'agit d'un arbre forestier pouvant atteindre 30 m de haut, à feuilles alternes, glabres, simples, coriaces, cunéiformes à la base, à pétiole caniculé et stipule caduque, limbe foliaire coriace, elliptique-lancéolé, atteignant 15 cm de long et 5-6 cm de large, cunée à la base, acuminé au sommet, penninerve, à 10-20 paires de nervures latérales. Son latex est blanc et ses fleurs sont à cyathiums groupés en petites cymes axillaires courtement pédonculées à subsessiles. Il présente des inflorescences en cyme, portant à chaque ramification un involucre de bractées enveloppant de très nombreuses fleurs mâles et une fleur femelle. Ses fruits sont des capsules profondément trilobées, contenant trois graines ovoïdes gris-jaunâtre tachetées de brun d'environ 15 mm de long et 20-25 mm de large.

*Anthostema senegalense* pousse dans les franges forestières au bord des rivières. L'espèce est endémique en Afrique Occidentale où elle se rencontre dans les galeries forestières en Guinée, Mali, Côte d'Ivoire, Liberia, Sénégal, Burkina Faso, Gambie, Guinée-Bissau, Sierra Leone, Bénin, et Nigeria. Peu répandue, cette plante est en voie d'extinction dans certains pays comme la Côte d'Ivoire du fait de la perturbation de son site naturel.

Pour réaliser la composition pharmaceutique selon l'invention, on utilise préférentiellement l'écorce de tronc d'*Anthostema senegalense,* de préférence après l'avoir fait sécher pour en éliminer le latex.

Bien que moins avantageuses, d'autres parties de la plante *Anthostema senegalense* pourraient également ou alternativement être utilisées pour réaliser la composition utilisée selon l'invention, telles que par exemple ses feuilles, ou son latex bien que plus toxique.

La composition pharmaceutique utilisée selon l'invention comprend préférentiellement un extrait végétal d'*Anthostema senegalense.* Cet extrait végétal est de préférence une solution végétale ou un extrait végétal liquide et par exemple selon sa concentration une teinture mère, une teinture officinale ou un extrait liquide d'*Anthostema senegalense.*

Cet extrait végétal d*'Anthostema senegalense* est un extrait polaire, par exemple aqueux, alcoolique ou hydro-alcoolique ; ou apolaire.

Cet extrait végétal est obtenu par tout moyen approprié et notamment par extraction, préférentiellement aqueuse, alcoolique ou hydro-alcoolique, par macération, infusion, décoction, percolation, digestion, lixiviation ou analogue.

Un extrait végétal polaire peut par exemple être obtenu par une extraction au méthanol ou à l'éthanol.

Un extrait végétal apolaire peut par exemple être obtenu par une extraction au dichlorométhane (CH₂Cl₂), au chloroforme (CHCl₃) ou à l'acétate d'éthyle (CH₃COOC₂H₅).

Un autre extrait végétal polaire peut par exemple être obtenu par percolation au méthanol à travers le marc issu de l'extraction au dichlorométhane.

Selon un mode de réalisation préférentiel de l'invention, on prépare un extrait végétal liquide à partir d'écorce de tronc d'*Anthostema senegalense* au moyen d'une extraction hydro-alcoolique par une technique de percolation avec agitation. Sa teneur en alcool est préférentiellement comprise entre 30 et 70°.

A titre d'exemple, on a préparé une teinture mère d'*Anthostema senegalense* avec une teneur en éthanol d'environ 70% V/V, à partir d'écorce sèche d'*Anthostema senegalense* par percolation exhaustive à froid suivie d'une concentration sous vide à l'évaporateur rotatif.

La composition utilisée selon l'invention peut adopter différentes formes galéniques, préférentiellement orales. Elle peut ainsi par exemple se présenter sous la forme d'infusettes contenant par exemple environ 5 grammes d'écorce de tronc d'*Anthostema senegalense* grossièrement broyée.

Elle peut également se présenter sous la forme de comprimés, préférentiellement orodispersibles, de capsules molles, de granules, de gélules, de poudre, d'ampoules, de sirop, de décocté, d'extrait fluide ou de toute autre forme galénique appropriée, préférentiellement orale, imaginable par l'homme du métier.

De préférence, la composition destinée à être utilisée selon l'invention est formulée sous la forme de microsphères, également appelées sphéroïdes, préférentiellement regroupées en gélules selon une quantité correspondant de préférence à une prise unitaire, par exemple journalière.

On bénéficie ainsi des nombreux avantages de cette forme galénique, à savoir sur le plan médical une libération rapide et une grande biodisponibilité des principes actifs, et sur le plan pratique une grande facilité d'utilisation pour le patient et une forme peu fragile et très stable à long terme même dans des conditions de stockage difficiles.

Ces microsphères peuvent être réalisées par différents procédés.

Un premier procédé consiste par exemple à réaliser une couche périphérique contenant l'extrait végétal d'*Anthostema senegalense* autour d'un noyau neutre, formé par exemple d'un mélange de sucre et d'amidon ou d'un cristal de sucre, de mannitol ou de sorbitol.

La couche contenant l'extrait végétal d'*Anthostema senegalense* peut avantageusement être réalisée par enrobage, imprégnation, pulvérisation ou projection, le principe actif étant pour cela préférentiellement mélangé à un agent de montage ou à un liant.

Selon un deuxième procédé préférentiel, les microsphères ou sphéroïdes peuvent être formés par extrusion et sphéronisation.

Pour cela, on commence par mélanger l'extrait végétal d'*Anthostema senegalense* à une substance absorbante et adsorbante, préférentiellement de type polymère naturel ou synthétique, présentant des propriétés plastiques compatibles avec les étapes d'extrusion et de sphéronisation de la suite du procédé.

Il s'agit par exemple de cellulose microcristalline, de cellulose microfine, d'un polymère cellulosique hydroxypropylé faiblement substitué, d'amidon, d'amidon modifié, de polysaccharides ou de toute autre substance ou mélange adapté.

Si le mélange est trop sec, un liquide d'humidification, aqueux ou non aqueux, peut également être ajouté pour obtenir une pâte homogène et malléable pouvant subir les étapes suivantes du procédé.

Le liquide d'humidification sert également de véhicule pour transporter et déposer le principe actif jusqu'au coeur de la substance absorbante et adsorbante, dans les microcavités du polymère.

Le procédé de fabrication consiste ensuite à extruder la masse humide à travers une filière à orifices calibrés. On obtient ainsi des filaments compacts, de section généralement cylindrique et définie, appelés « extrudats ».

Ces « extrudats » sont ensuite placés dans un appareil cylindrique appelé « sphéroniseur » contenant en partie inférieure un disque cannelé tournant à une vitesse variable et contrôlée. Sous l'effet de la force centrifuge exercée par la rotation du disque tournant, les « extrudats » se fragmentent régulièrement, puis se transforment en sphères par un effet de roulage-liage. On obtient ainsi les sphéroïdes souhaités.

Ces deux procédés peuvent comprendre en outre au moins une étape de séchage et/ou une étape de calibrage.

Le séchage peut se faire par chauffage léger par exemple à une température comprise entre 30 et 40°C ou par une simple exposition à l'air libre pendant une durée suffisante.

Enfin, les sphéroïdes résultants peuvent, de manière optionnelle, être revêtus en périphérie d'une pellicule extérieure en vue de les protéger par exemple de l'humidité extérieure, de la chaleur, des conditions agressives de l'organisme ou à effet retard.

En plus de l'extrait végétal d'*Anthostema senegalense,* la composition destinée à être utilisée selon l'invention peut comprendre en outre un ou plusieurs autres composés ou excipients appropriés, par exemple choisis parmi les extraits d'une ou plusieurs autres plantes, les vitamines, les minéraux, les oligoéléments, les arômes, les agents de montage, les composés liants, les composés glissants, les composés lubrifiants, les tensioactifs, les sucres, le lactose, le sorbitol, le mannitol, les amidons ou amidons modifiés, les maltodextrines, les carbonates, les citrates, la gélatine, la polyvinylpyrrolidone, les polysaccharides, les dérivés cellulosiques, la carboxyméthylcellulose sodique réticulée, la cellulose microcristalline, la cellulose microfine, les polymères cellulosiques hydroxypropylés faiblement substitués ou tout autre composé approprié.

Elle pourra comprendre également un ou plusieurs composés augmentant l'efficacité de l*'Anthostema senegalense,* le protégeant ou aidant à sa libération rapide.

Elle pourra également comprendre en association une ou plusieurs autres substances antivirales ou antirétrovirales connues, notamment une ou plusieurs de celles utilisées dans le cadre des traitements classiques contre le sida (trithérapies ou autres). La composition selon l'invention pourra ainsi comprendre par exemple, en plus de l'*Anthostema senegalense,* de la zidovudine ou de l'azidothymidine (AZT), afin de renforcer son activité anti-VIH.

Les exemples suivants permettront de mieux illustrer l'invention :

### Exemple 1 :

Des sphéroïdes ont été réalisés par le procédé d'extrusion et de sphéronisation décrit précédemment, à partir d'un extrait hydro-alcoolique d'écorce de tronc d'*Anthostema senegalense,* préparé selon la pharmacopée européenne, avec une teneur en éthanol de 70% V/V et un résidu sec d'environ 10 %. Ces sphéroïdes ont été rassemblés en gélules de 350 mg.
Pour une gélule de 350 mg :

| | |
|---|---|
| • extrait *d'Anthostema senegalense* | 350 ml équivalent résidu sec 35 mg |
| • microcellulose | q.s. |

Les sphéroïdes obtenus ont été regroupés en gélules de 350 mg correspondant à une dose unitaire de composition, pour une posologie préférentielle de trois gélules par jour.

### Exemple 2 :

La prise de vitamines et de minéraux étant bénéfique pour l'amélioration de l'état de santé général des personnes immunodéprimées, des sphéroïdes contenant un mélange de vitamines et de sélénium en plus de l'extrait de plante ont été préparés.

Ces sphéroïdes ont été réalisés comme précédemment à partir de l'extrait hydro-alcoolique décrit d'écorce de tronc de d'*Anthostema senegalense.*

Avant l'extrusion et la sphéronisation, on a toutefois rajouté à cet extrait des vitamines et du sélénium dans les proportions suivantes :
Pour 2,1 kg d'extrait :

| | |
|---|---|
| vitamine PP : | 70 g |
| vitamine B1 : | 8 g |
| vitamine B2 : | 8 g |
| vitamine B6 : | 8 g |
| sélénium : | 48 mg |

Les sphéroïdes obtenus ont été rassemblés en gélules de 350 mg.

Afin de démontrer les propriétés anti-VIH de la composition destinée à être utilisée selon l'invention, les présents inventeurs ont mené deux études : une série de tests in vitro et un essai clinique qui ont permis de conclure à l'efficacité d'une composition à base d'*Anthostema senegalense.* Les résultats de ces études sont résumés ci-dessous.

### Tests in vitro :

Un extrait apolaire et un extrait polaire d'*Anthostema senegalense* ont été préparés par extraction respectivement au dichlorométhane et au méthanol à partir d'écorce de tronc séchée d'*Anthostema senegalense.*

L'efficacité de ces extraits contre le virus du VIH a été testée in vitro par la mesure de la concentration inhibitrice médiane (IC₅₀) de ces substances sur le virus VIH de type 1 (souche III_{B}) et sur le virus de type 2 (souche ROD). Leur index de sélectivité a également été mesuré dans chaque cas.

Afin de servir de témoin, trois molécules antirétrovirales connues, utilisées dans le traitement de l'infection par le VIH : la névirapine, la didéoxycitidine et la didéoxyinosine, ont également été testées dans les mêmes conditions à titre de comparaison.

Les résultats obtenus ont été rassemblés dans le tableau ci-dessous :

| | **VIH-1 Souche III_{B}** | | **VIH-2 Souche ROD** | |
|---|---|---|---|---|
| **Echantillon testé** | **IC₅₀ (µg/ml)** | **Index de sélectivité** | **IC₅₀ (µg/ml)** | **Index de sélectivité** |
| *Anthostema senegalense* extrait apolaire (CH₂Cl₂) | 0,250 | 119 | 0,0519 | 714 |
| *Anthostema senegalense* extrait polaire (MeOH) | 2,04 | 24 | 0,42 | 132 |
| Nevirapine | 0,047 | | > 4,00 | |
| Didéoxycitidine | 0,29 | | 0,30 | |
| Didéoxyinosine | 2,89 | | 4,59 | |

On constate que les extraits polaire et apolaire d'*Anthostema senegalense* exercent in vitro une inhibition significative de chacun des deux types de VIH (VIH-1 et VIH-2) testés et ce avec un index de sélectivité satisfaisant.

L'extrait apolaire présente une activité inhibitrice très prononcée à l'encontre du VIH-1, comparable à celle de la didéoxycitidine et meilleure que celle de la didéoxyinosine. Cette activité est encore meilleure à l'encontre du VIH-2 pour lequel elle est nettement meilleure que celle de la névirapine, de la didéoxycitidine et de la didéoxyinosine.

L'extrait polaire présente une activité inhibitrice prononcée à l'encontre du VIH-1, un peu moins bonne que celle de la névirapine et de la didéoxycitidine mais légèrement meilleure que celle de la didéoxyinosine, et très prononcée à l'encontre du VIH-2 pour lequel elle est comparable à celle de la didéoxycitidine et nettement meilleure que celle de la névirapine et de la didéoxyinosine.

### Essai clinique :

Six patients séropositifs « naïfs », c'est-à-dire n'ayant jamais pris de traitement antirétroviral contre le VIH, ont été traités pendant plusieurs mois avec un extrait aqueux d'*Anthostema senegalense* (patient 5) ou un extrait hydro-alcoolique d'*Anthostema senegalense* (patients 2, 3 et 4) ou encore avec des infusettes contenant de l'écorce de tronc séchée d'*Anthostema senegalense* (patients 1 et 6).

L'extrait aqueux a été préparé par décoction pendant 15 minutes de 500 g d'écorce séchée et broyée d'*Anthostema senegalense* dans 1 litre d'eau, puis filtration. Un demi-verre à eau (soit environ 10 cl) de ce décocté a été donné chaque jour aux patients traités par l'extrait aqueux.

L'extrait hydro-alcoolique a été préparé à température ambiante par percolation de 1 kg d'écorce séchée et broyée d'*Anthostema senegalense,* puis par concentration de l'extrait obtenu jusqu'à obtenir un litre d'extrait hydro-alcoolique fluide. Une cuillère à café (soit environ 5 ml) de cet extrait fluide a été donnée chaque jour aux patients traités par l'extrait hydro-alcoolique.

Pour les infusettes, 5 g d'écorce séchée et broyée d'*Anthostema senegalense* ont été placés dans chaque infusette. Les patients traités par infusettes en ont pris une par jour.

Le taux de cellules CD4 de ces patients, correspondant au nombre de cellules CD4 par mm³ de sang et reflétant le nombre de lymphocytes T4 du patient, a été mesuré tous les mois pendant toute la durée de l'étude.

Les résultats obtenus ont été regroupés dans le tableau suivant :

| | **Taux de CD4** | | | | | | |
|---|---|---|---|---|---|---|---|
| | Patient 1 F, 32 ans infusettes | Patient 2 F, 23 ans extrait hydro-alcoolique | Patient 3 M, 46 ans extrait hydro-alcoolique | Patient 4 F, 24 ans extrait hydro-alcoolique | Patient 5 M, 48 ans extrait aqueux | Patient 6 F, 30 ans infusettes | Moyenne ± SD |
| Mois 1 | 134 | 118 | 276 | 142 | 218 | 110 | 166 ± 66 |
| Mois 2 | 186 | 120 | 290 | 178 | 200 | 124 | 183 ± 62 |
| Mois 3 | 180 | 140 | 325 | 132 | 264 | 116 | 192 ± 84 |
| Mois 4 | 128 | 205 | 380 | 180 | 280 | 102 | 213 ± 103 |
| Mois 5 | 150 | 280 | | 215 | | | 215 ± 65 |
| Mois 6 | 162 | 200 | | 230 | | | 197 ± 34 |
| Mois 7 | 180 | 290 | | | | | 235 ±78 |

On constate que le traitement par la composition utilisée selon l'invention a permis d'améliorer le taux des cellules CD4 chez cinq patients sur six au bout de quatre à sept mois de suivi. Leur ratio de cellules CD4/CD8 a également été amélioré.

En outre, on a également noté chez ces patients une amélioration et même une disparition, de certaines manifestations cliniques de l'infection par le VIH :
La composition selon l'invention a ainsi permis de traiter :
- des diarrhées chroniques chez trois patients ;
- des dermatoses chez deux patients ;
- des allergies dermiques chez quatre patients.

La disparition de ces manifestations cliniques de l'infection par le VIH, grâce à la prise de la composition utilisée selon l'invention, a permis de considérablement améliorer la qualité de vie de ces patients.

On constate donc, en plus d'une réduction de l'infection par le virus du VIH se traduisant par une augmentation du taux des cellules CD4, une amélioration de l'état de santé général des patients liée à la disparition de manifestations cliniques qui accompagnent l'infection par le virus du VIH.

Les présents inventeurs ont également remarqué que la composition à base d'*Anthostema senegalense* utilisée selon l'invention présente également des propriétés antifongiques, notamment contre *Candida albicans,* champignon responsable de la candidose qui est une maladie fréquemment rencontrée chez les patients infectés par le virus du VIH.

Des études biologiques in vitro ont ainsi permis de montrer que l'extrait polaire au méthanol d'écorce de tronc d'*Anthostema senegalense* présente une activité notable à l'égard de *Candida albicans* avec une concentration inhibitrice médiane (IC₅₀) égale à 29,71 µg/ml, conférant ainsi à la composition selon l'invention un potentiel thérapeutique intéressant pour le traitement des candidoses.

On constate encore une fois que la composition à base d'*Anthostema senegalense* utilisée selon l'invention permet en outre avantageusement de traiter les manifestations cliniques qui accompagnent l'infection par le virus du VIH.

Cette composition offre une alternative efficace et beaucoup moins chère que les trithérapies actuelles, pour le traitement de l'infection par le virus du VIH, du sida et des manifestations cliniques qui l'accompagnent.

Comme exposé précédemment, elle peut en outre être proposée sous une forme galénique qui ne pose pas de problèmes de conservation.

Pour toutes ces raisons, la composition utilisée selon l'invention apparait comme une solution thérapeutique extrêmement prometteuse pour les pays en voie de développement.

De manière évidente, l'invention ne se limite pas aux modes de réalisation préférentiels décrits précédemment, l'homme du métier pouvant y apporter de nombreuses modifications et imaginer d'autres variantes sans sortir ni de la portée, ni du cadre de l'invention définis par les revendications.

## Revendications

1. Composition pharmaceutique à base d'*Anthostema senegalense* pour son utilisation comme médicament dans le traitement de l'infection par le Virus de l'Immunodéficience Humaine (VIH), du sida et des manifestations cliniques qui l'accompagnent.

2. Composition pharmaceutique à base d'*Anthostema senegalense* pour son utilisation selon la revendication 1 comme médicament antirétroviral contre le virus VIH de type 1 ou de type 2.

3. Composition pharmaceutique à base d'*Anthostema senegalense* pour son utilisation selon l'une quelconque de revendications précédentes **caractérisée en ce qu'**elle est obtenue à partir d'écorce de tronc *d'Anthostema senegalense.*

4. Composition pharmaceutique à base d'*Anthostema senegalense* pour son utilisation selon la revendication 3 **caractérisée en ce qu'**elle est obtenue à partir d'écorce de tronc séchée.

5. Composition pharmaceutique à base d'*Anthostema senegalense* pour son utilisation selon l'une quelconque des revendications précédentes **caractérisée en ce qu'**elle comprend un extrait végétal *d'Anthostema senegalense.*

6. Composition pharmaceutique à base d'*Anthostema senegalense* pour son utilisation selon la revendication 5 **caractérisée en ce que** l'extrait végétal d'*Anthostema senegalense* est un extrait polaire, aqueux, alcoolique ou hydro-alcoolique.

7. Composition pharmaceutique à base d'*Anthostema* senegalense pour son utilisation selon la revendication 6 **caractérisée en ce que** l'extrait végétal d'*Anthostema senegalense* est une teinture mère d'*Anthostema senegalense* avec une teneur en éthanol d'environ 70% V/V.

8. Composition pharmaceutique à base d'*Anthostema* senegalense pour son utilisation selon la revendication 5 **caractérisée en ce que** l'extrait végétal d'*Anthostema senegalense* est un extrait apolaire.

9. Composition pharmaceutique à base d'*Anthostema senegalense* pour son utilisation selon l'une quelconque de revendications précédentes **caractérisée en ce qu'**elle comprend en outre un ou plusieurs composés ou excipients choisis parmi les extraits de plantes, les vitamines, les minéraux, les oligoéléments, les arômes, les agents de montage, les composés liants, les composés glissants, les composés lubrifiants, les tensioactifs, les sucres, le lactose, le sorbitol, le mannitol, les amidons et amidons modifiés, les maltodextrines, les carbonates, les citrates, la gélatine, la polyvinylpyrrolidone, les polysaccharides, les dérivés cellulosiques, la carboxyméthylcellulose sodique réticulée, la cellulose microcristalline, la cellulose microfine, les polymères cellulosiques hydroxypropylés faiblement substitués, et les substances antivirales ou antirétrovirales.

10. Composition pharmaceutique à base d'*Anthostema senegalense* pour son utilisation selon la revendication 9 **caractérisée en ce qu'**elle comprend en outre de la zidovudine, de l'azidothymidine, de la vitamine PP, de la vitamine B1, de la vitamine B2, de la vitamine B6 ou du sélénium.

11. Composition pharmaceutique à base d'*Anthostema senegalense* pour son utilisation selon l'une quelconque de revendications précédentes **caractérisée en ce qu'**elle se présente sous une forme galénique orale.

12. Composition pharmaceutique à base d'*Anthostema senegalense* pour son utilisation selon la revendication 11 **caractérisée en ce qu'**elle se présente sous une forme galénique d'infusettes, de microsphères, de comprimés, de comprimés orodispersibles, de capsules molles, de granules, de gélules, de poudre, d'ampoules, de sirop, de décocté ou d'extrait fluide.

13. Composition pharmaceutique à base d'*Anthostema senegalense* pour son utilisation selon la revendication 12 **caractérisée en ce qu'**elle se présente sous une forme galénique d'infusettes contenant environ cinq grammes d'écorce de tronc d'*Anthostema senegalense* grossièrement broyée.

14. Composition pharmaceutique à base d'*Anthostema senegalense* pour son utilisation selon la revendication 12 **caractérisée en ce qu'**elle se présente sous une forme galénique de microsphères réalisées par un procédé d'extrusion et de sphéronisation.

15. Composition pharmaceutique à base d'*Anthostema senegalense* pour son utilisation selon la revendication 14 **caractérisée en ce que** les microsphères sont regroupées en gélules.

## Patentansprüche

1. Auf *Anthostema senegalense* basierende pharmazeutische Zusammensetzung zur Verwendung als Arzneimittel bei der Behandlung der Infektion mit dem humanen Immumdefizienz-Virus (HIV), von AIDS und der begleitenden klinischen Erscheinungsformen.

2. Auf *Anthostema senegalense* basierende pharmazeutische Zusammensetzung zur Verwendung nach Anspruch 1 als antiretrovirales Arzneimittel gegen den Typ-1 oder Typ-2 HIV-Virus.

3. Auf *Anthostema senegalense* basierende pharmazeutische Zusammensetzung zur Verwendung nach irgendeinem der vorhergehenden Ansprüche, **dadurch gekennzeichnet, dass** sie aus Stammrinde von *Anthostema senegalense* erhalten wird.

4. Auf *Anthostema senegalense* basierende pharmazeutische Zusammensetzung zur Verwendung nach Anspruch 3, **dadurch gekennzeichnet, dass** sie aus getrockneter Stammrinde erhalten wird.

5. Auf *Anthostema senegalense* basierende pharmazeutische Zusammensetzung zur Verwendung nach irgendeinem der vorhergehenden Ansprüche, **dadurch gekennzeichnet, dass** sie einen Pflanzenextrakt von *Anthostema senegalense* umfasst.

6. Auf *Anthostema senegalense* basierende pharmazeutische Zusammensetzung zur Verwendung nach Anspruch 5, **dadurch gekennzeichnet, dass** der Pflanzenextrakt von *Anthostema senegalense* ein polarer, wässriger, alkoholischer oder hydroalkoholischer Extrakt ist.

7. Auf *Anthostema senegalense* basierende pharmazeutische Zusammensetzung zur Verwendung nach Anspruch 6, **dadurch gekennzeichnet, dass** der Pflanzenextrakt von *Anthostema senegalense* eine Urtinktur von *Anthostema senegalense* mit einem Ethanolgehalt von etwa 70% V/V ist.

8. Auf *Anthostema senegalense* basierende pharmazeutische Zusammensetzung zur Verwendung nach Anspruch 5, **dadurch gekennzeichnet, dass** der Pflanzenextrakt von *Anthostema senegalense* ein unpolarer Extrakt ist.

9. Auf *Anthostema senegalense* basierende pharmazeutische Zusammensetzung zur Verwendung nach irgendeinem der vorhergehenden Ansprüche, **dadurch gekennzeichnet, dass** sie zusätzlich eine oder mehrere Verbindungen oder Hilfsstoffe enthält, die aus Pflanzenextrakten, Vitaminen, Mineralstoffen, Spurenelementen, Aromen, Eindeckmitteln, Bindemitteln, Gleitverbindungen, Schmierverbindungen, Tensiden, Zuckern, Laktose, Sorbitol, Mannitol, Stärken und modifizierten Stärken, Maltodextrinen, Karbonaten, Citraten, Gelatine, Polyvinylpyrrolidon, Polysacchariden, Cellulosederivaten, vernetzter Natriumcarboxymethylcellulose, mikrokristalliner Cellulose, mikrofeiner Cellulose, schwach substituierten hydroxypropylierten Cellulosepolymeren und den antiviralen oder antiretroviralen Substanzen ausgewählt sind.

10. Auf *Anthostema senegalense* basierende pharmazeutische Zusammensetzung zur Verwendung nach Anspruch 9, **dadurch gekennzeichnet, dass** sie zusätzlich Zidovudin, Azidothymidin, Vitamin PP, Vitamin B1, Vitamin B2, Vitamin B6 oder Selen umfassst.

11. Auf *Anthostema senegalense* basierende pharmazeutische Zusammensetzung zur Verwendung nach irgendeinem der vorhergehenden Ansprüche, **dadurch gekennzeichnet, dass** sie in oraler galenischer Form vorliegt.

12. Auf *Anthostema senegalense* basierende pharmazeutische Zusammensetzung zur Verwendung nach Anspruch 11, **dadurch gekennzeichnet, dass** sie in galenischer Form von Teebeuteln, Mikrosphären, Tabletten, Schmelztabletten, Weichkapseln, Granulaten, Kapseln, Pulver, Ampullen, Sirup, Dekokt oder flüssigem Extrakt vorliegt.

13. Auf *Anthostema senegalense* basierende pharmazeutische Zusammensetzung zur Verwendung nach Anspruch 12, **dadurch gekennzeichnet, dass** sie in galenischer Form von Teebeuteln vorliegt, die etwa fünf Gramm grob zerkleinerte Stammrinde von *Anthostema senegalense* enthalten.

14. Auf *Anthostema senegalense* basierende pharmazeutische Zusammensetzung zur Verwendung nach Anspruch 12, **dadurch gekennzeichnet, dass** sie in galenischer Form von Mikrosphären vorliegt, die durch ein Extrusions- und Sphäronisierungsverfahren hergestellt werden.

15. Auf *Anthostema senegalense* basierende pharmazeutische Zusammensetzung zur Verwendung nach Anspruch 14, **dadurch gekennzeichnet, dass** die Mikrosphären zu Kapseln gruppiert sind.

## Claims

1. A pharmaceutical composition based on *Anthostema senegalense* for its use as a drug in the treatment of the infection with the Human Immunodeficiency Virus (HIV), AIDS and the accompanying clinical manifestations.

2. The pharmaceutical composition based on *Anthostema senegalense* for its use according to claim 1 as an antiretroviral drug for type 1 or type 2 HIV virus.

3. The pharmaceutical composition based on *Anthostema senegalense* for its use according to any one of the preceding claims, wherein it is obtained from stem bark of *Anthostema senegalense.*

4. The pharmaceutical composition based on *Anthostema senegalense* for its use according to claim 3, wherein it is obtained from dried stem bark.

5. The pharmaceutical composition based on *Anthostema senegalense* for its use according to any one of the preceding claims, wherein it comprises a plant extract of *Anthostema senegalense.*

6. The pharmaceutical composition based on *Anthostema senegalense* for its use according to claim 5, wherein the plant extract of *Anthostema senegalense* is a polar, aqueous, alcoholic or hydro-alcoholic extract.

7. The pharmaceutical composition based on *Anthostema senegalense* for its use according to claim 6, wherein the plant extract of *Anthostema senegalense* is a mother tincture of *Anthostema senegalense* with an ethanol content of about 70% V/V.

8. The pharmaceutical composition based on *Anthostema senegalense* for its use according to claim 5, wherein the plant extract of *Anthostema senegalense* is an apolar extract.

9. The pharmaceutical composition based on *Anthostema senegalense* for its use according to any one of the preceding claims, wherein it comprises in addition one or several compounds or excipients chosen from extracts from plant extracts, vitamins, minerals, trace elements, aromas, mounting agents, binding compounds, sliding compounds, lubricating compounds, surfactants, sugars, lactose, sorbitol, mannitol, starches and modified starches, maltodextrins, carbonates, citrates, gelatin, polyvinylpyrrolidone, polysaccharides, cellulose derivatives, crosslinked sodium carboxymethylcellulose, microcrystalline cellulose, micro-fine cellulose, weakly substituted hydroxypropylated cellulosic polymers, and the antiviral or antiretroviral substances.

10. The pharmaceutical composition based on *Anthostema senegalense* for its use according to claim 9, wherein it comprises in addition zidovudine, azidothymidine, vitamin PP, vitamin B1, vitamin B2, vitamin B6 or selenium.

11. The pharmaceutical composition based on *Anthostema senegalense* for its use according to any one of the preceding claims, wherein it is in an oral galenic form.

12. The pharmaceutical composition based on *Anthostema senegalense* for its use according to claim 11, wherein it is in a galenic form of infusettes, microspheres, tablets, orodispersible tablets, soft capsules, granules, capsules, powder, ampoules, syrup, decoction or fluid extract.

13. The pharmaceutical composition based on *Anthostema senegalense* for its use according to claim 12, wherein it is in a galenic form of infusettes containing about five grams of coarsely crushed stem bark of *Anthostema senegalense.*

14. The pharmaceutical composition based on *Anthostema senegalense* for its use according to claim 12, wherein it is in a galenic form of microspheres produced by an extrusion and spheronization method.

15. The pharmaceutical composition based on *Anthostema senegalense* for its use according to claim 14, wherein the microspheres are grouped into capsules.
